Europäisches Patentamt

European Patent Office

Office européen des brevets

(11) Veröffentlichungsnummer : **0 050 255
B1**

# EUROPÄISCHE PATENTSCHRIFT

(45) Veröffentlichungstag der Patentschrift :
07.11.84

(21) Anmeldenummer : 81107895.5

(22) Anmeldetag : 03.10.81

(51) Int. Cl.³ : **A 61 J 1/00, B 65 B 11/58,
B 65 B 31/02**

(54) **Verfahren zum Verpacken von Parenteralia in dampfsterilisierbaren, klarsichtigen Behältern aus weichen Kunststoffen.**

(30) Priorität : 16.10.80 DE 3039093

(43) Veröffentlichungstag der Anmeldung :
28.04.82 Patentblatt 82/17

(45) Bekanntmachung des Hinweises auf die Patenterteilung : 07.11.84 Patentblatt 84/45

(84) Benannte Vertragsstaaten :
AT BE CH DE FR GB IT LI NL SE

(56) Entgegenhaltungen :
CH-A- 444 382
DE-A- 2 933 071
FR-A- 2 108 380

(73) Patentinhaber : Biotest-Serum-Institut GmbH
Flughafenstrasse 4
D-6000 Frankfurt-Niederrad (DE)

(72) Erfinder : Eichentopf, Bertram, Dr.
Im Lauer 7
D-6232 Bad Soden (DE)

(74) Vertreter : Beil, Walter, Dr. et al
BEIL, WOLFF & BEIL Rechtsanwälte Adelonstrasse 58
D-6230 Frankfurt am Main 80 (DE)

## Beschreibung

Die Erfindung bezieht sich auf ein im Oberbegriff des Anspruchs 1 angegebenes Verfahren zum Verpacken von Parenteralia in dampfsterilisierbaren, klarsichtigen Behältern aus weichgemachten Kunststoffen.

Infusionslösungen werden seit geraumer Zeit nicht nur in Glasflaschen, sondern auch in Kunststoffbehältnisse abgefüllt. Geeignete Materialien für derartige Behälter sind beispielsweise Polyethylen, Polypropylen und Polyvinylchlorid. Die beiden erstgenannten Materialien sind relativ starr, nicht ganz klarsichtig und im Falle des Polyethylens auch nicht, wie üblich, bei 121 °C dampfsterilisierbar. Weichgemachtes Polyvinylchlorid für medizinische Zwecke, z. B. ein mit Dioctylphthalat weichgemachtes Polyvinylchlorid, erfüllt in fast idealer Weise alle Anforderungen an ein Material zur Fertigung von Infusionslösungsbehältnissen.

Nachteil des Polyvinylchlorids ist seine geringe Wasserdampfsperre, so daß Lösungen, die in Polyvinylchlorid-Beutel abgefüllt werden, zusätzlich durch eine zweite Hülle gegen zu hohe Wasserverdunstung geschützt werden müssen, um ausreichende Lagerzeiten garantieren zu können.

Üblicherweise werden zu diesem Zweck luftdicht verschweißte Polyethylen-Tüten verwendet. Da dieses Material aber nicht dampfsterilisierbar ist, muß zunächst die Lösung im nackten Polyvinylchlorid-Beutel sterilisiert werden, um anschließend endgültig verpackt zu werden. Neben erhöhtem Arbeitsaufwand ist hierbei die Gefahr von Versporung oder Verpilzung des Innern des Polyethylen-Schutzbeutels gegeben, insbesondere bei Verwendung geleimter Papieretiketten auf dem Polyvinylchlorid-Innenbeutel. Es sind auch bereits seit längerer Zeit dampfsterilisierbare Außenverpackungen und diverse Materialien zu ihrer Fertigung bekannt. Üblicherweise werden für diesen Zweck Laminate aus Polyester und Polypropylen verwendet, die eine hohe Wasserdampfsperre aufweisen und so den Innenbeutel vor erhöhtem Wasserverlust schützen können. Probleme bei der Verwendung solcher Schutzhüllen ergeben sich jedoch dadurch, daß während der Dampfsterilisation Wasser aus dem Polyvinylchlorid-Beutel in den Raum zwischen Innenbeutel und Schutzhülle austritt und das Polyvinylchlorid eintrübt, wodurch ein wesentlicher Vorteil dieses Materials, nämlich die Klarsichtigkeit, die eine Sichtkontrolle der Infusionslösung ermöglicht, nicht mehr gegeben ist. Das Wasser, das einmal aus dem inneren Behältnis ausgetreten ist, kann aufgrund der hohen Wasserdampfsperre der Außenhülle nur sehr langsam verdunsten, so daß solcherart verpackte und dampfsterilisierte Beutel auch über einen Zeitraum von 2 Jahren eingetrübt bleiben und der Inhalt sich so der Sichtkontrolle durch den Anwender entzieht, selbst bei entfernter Außenhülle.

Die Menge Wasser, die während eines normalen Sterilisationszyklus in den Raum zwischen Innen- und Außenbeutel gelangt, ist beträchtlich und beträgt etwa 0,5 g bei einem Infusionslösungsvolumen von 500 ml. Aus der DE-A-29 33 071 ist bekannt, daß man die Eintrübung des Polyvinylchlorid-Materials verhindern kann, indem man einen zusätzlichen Behälter aus Papier, der mit einem Trocknungsmittel gefüllt ist, mit in den Raum zwischen Infusionslösungsbeutel und Außenhülle einschweißt. Gegen ein solches Verfahren sprechen jedoch neben dem zusätzlichen Aufwand Bedenken gegen das Trockenmittel, insbesondere bei Verletzung der Verpackung.

Der Erfindung liegt die Aufgabe zugrunde, Parenteralia in klarsichtigen Behältern aus weichen Kunststoffen auf einfache Weise derart zu verpacken, daß die Behälter auch nach Dampfsterilisation klarsichtig bleiben oder höchstens vorübergehend kurzzeitig eingetrübt werden und somit der geforderten visuellen Kontrolle zugänglich sind, ohne einen Fremdkörper wie ein Trockenmittel zwischen Innenbehälter und Schutzhülle einzubringen.

Diese Aufgabe wird ausgehend von einem Verfahren der im Oberbegriff des Anspruchs 1 genannten Art erfindungsgemäß dadurch gelöst, daß man den gefüllten und verschlossenen Innenbehälter in eine entsprechend seinen Konturen tiefgezogene Unterbahn der Schutzhülle legt und mit der Oberbahn der Schutzhülle bedeckt, an die Schutzhülle ein Vakuum anlegt und die Ränder von Unterbahn und Oberbahn der Schutzhülle miteinander verschweißt.

Überraschenderweise zeigte sich, daß eine erfindungsgemäß erhaltene Verpackung ohne bleibende Eintrübung des Innenbehälters bei 121 °C dampfsterilisiert werden konnte, und zwar in der üblichen Sterilisationszeit von 20 Minuten.

Bei der Durchführung des erfindungsgemäßen Verfahrens wird zunächst der Innenbehälter mit der Infusionslösung gefüllt und verschlossen. Der kollabierbare Innenbehälter besteht aus durchsichtigem weichen Kunststoff. Ein bevorzugtes Material ist ein mit Dioctylphthalat weichgemachtes Polyvinylchlorid. Sodann wird der mit der Infusionslösung gefüllte und verschlossene Beutel, der ein Fassungsvermögen von 100 bis 3 000 ml aufweisen kann, in eine genau seinen Konturen entsprechend tiefgezogene Unterbahn der Schutzhülle gelegt und mit der Oberbahn der Schutzhülle bedeckt.

Als Material für die Schutzhülle werden solche durchsichtigen, weichen Kunststoffe verwendet, die für Mikroorganismen undurchdringlich sind, eine hohe Wasserdampfsperre aufweisen und bei 121 °C sterilisierbar sind. Bevorzugt werden Laminate eingesetzt, beispielsweise Laminate aus Polyester und Polypropylen, wie sie unter der Handelsbezeichnung « Flexovac V 5163 » im Handel erhältlich sind, oder aus Polyamid und Polypropylen, wie sie unter den Handelsbezeichnungen « Nyloflex » oder « Polyathen » erhältlich sind, oder auch aus Polyethylenterephthalat und Polypropylen, wie sie unter der Handelsbezeichnung « Ibetherm » erhältlich sind. Ober- und Unterbahn können aus unterschiedlichem Material hergestellt werden und eine unterschiedliche Stärke aufweisen. Bedingung ist jedoch, daß die innenliegenden Schichten der Laminate miteinander verschweißbar, sind. Wenn nur die Unterbahn tiefgezogen wird, sollte die Unterbahn vorzugsweise etwas stärker als die Oberbahn sein. Beispielsweise kann eine Oberbahn aus einem 52 μm starken Laminat aus Polyethylenterephthalat und Polypropylen, dessen Polypropylenschicht eine Stärke von 40 μm aufweist, mit einer Unterbahn aus einem 110 μm starken Laminat aus Polyamid und Polypropylen verschweißt werden, dessen Polypropylenschicht eine Stärke von 50 μm aufweist.

Nach dem Auflegen der Oberbahn auf den gefüllten Infusionslösungsbeutel und die Unterbahn der Schutzhülle wird an die Schutzhülle ein Vakuum angelegt, das vorzugsweise etwa 500 bis 600 mbar betragen sollte. Gleichzeitig oder unmittelbar anschließend werden die Ränder von Oberbahn und Unterbahn der Schutzhülle miteinander verschweißt. Beim Anlegen des Vakuums legt sich die Folie der Schutzhülle so dicht an die Folie des Innenbehälters an, daß ein dichter Verbund entsteht. Durch diesen dichten Verbund wird überraschenderweise erreicht, daß keine bleibende Eintrübung des Innenbehälters auftritt, wenn die gefüllte Verpackung anschließend dampfsterilisiert wird. Es kann zwar direkt im Anschluß an die Sterilisation eine geringe vorübergehende Eintrübung auftreten, diese ist jedoch nach spätestens drei bis vier Tagen, die ohnehin als Wartezeit für die vorgeschriebenen Kontrollen benötigt werden, wieder verschwunden. Die auf diese Weise erhaltene klarsichtige Verpackung ist in hervorragender Weise einer Sichtkontrolle zugänglich. Eine erneute Eintrübung erfolgt auch bei längerer Lagerung nicht.

Ein weiterer Vorteil der erfindungsgemäß erhältlichen Verpackung liegt darin, daß bei Verletzungen der Schutzhülle, die zu Undichtigkeiten führen, die Schutzhülle dem Innenbehälter nicht mehr bündig anliegt, so daß eine noch so geringfügige Undichtigkeit sofort deutlich sichtbar wird.

Die Erfindung wird durch das folgende Beispiel näher erläutert.

Beispiel

Klarsichtige Infusionslösungsbeutel mit einem Fassungsvermögen von 500 ml aus mit Dioctylphthalat weichgemachtem Polyvinylchlorid für medizinische Zwecke mit einer Stärke von 350 μm wurden mit einer Infusionslösung eines Plasmaersatzmittels, d. h. einer 5,5 %igen Lösung von Oxypolygelatine in physiologischer Elektrolytlösung, gefüllt und verschlossen. Jeder gefüllte und verschlossene Polyvinylchlorid-Beutel wurde in eine tiefgezogene Unterbahn einer Schutzhülle gelegt, deren Konturen genau denen des gefüllten Beutels entsprachen. Die Unterbahn bestand aus einem Laminat aus Polyester und Polypropylen mit einer Gesamtstärke von 125 μm, wobei die Stärke der inneren Polyesterschicht 50 μm betrug (erhältlich unter der Handelsbezeichnung « Flexovac V 5163 »). Sodann wurden Beutel und Unterbahn der Schutzhülle mit der Oberbahn der Schutzhülle bedeckt, die ebenfalls aus einem Laminat aus Polyester und Polypropylen bestand. Die Oberbahn hatte eine Gesamtstärke von 80 μm, wobei die Stärke der inneren Polyesterschicht 30 μm betrug.

Anschließend wurde ein Vakuum von 600 mbar an die Schutzhülle angelegt, und Ober- und Unterbahn der Schutzhülle wurden ringsherum an den Rändern miteinander verschweißt.

Die fertig versiegelten Verpackungen ließen sich einer üblichen Wasserdampfsterilisation im Autoklaven bei 121 °C für die Dauer von 20 Minuten unterwerfen, ohne daß die Polyvinylchlorid-Beutel sich bleibend eintrübten. Auch nach längerer Lagerung blieb die gesamte Verpackung klarsichtig und war einer Sichtkontrolle gut zugänglich.

**Ansprüche**

1. Verfahren zum Verpacken von Parenteralia in dampfsterilisierbaren, klarsichtigen Behältern aus weichen Kunststoffen, wobei die Parenteralia zunächst in einen Innenbehälter aus durchsichtigem, weichem Kunststoff abgefüllt und dann der Innenbehälter verschlossen und in einer Schutzhülle aus durchsichtigem, weichem, für Mikroorganismen undurchdringlichem Kunststoff mit hoher Wasserdampfsperre versiegelt wird, dadurch gekennzeichnet, daß man den gefüllten und verschlossenen Innenbehälter in eine entsprechend seinen Konturen tiefgezogene Unterbahn der Schutzhülle legt und mit der Oberbahn der Schutzhülle bedeckt, an die Schutzhülle ein Vakuum anlegt und die Ränder von Unterbahn und Oberbahn der Schutzhülle miteinander verschweißt.

2. Verfahren nach Anspruch 1, dadurch gekennzeichnet, daß man vor oder während der Versiegelung der Schutzhülle ein Vakuum von etwa 500 bis 600 mbar anwendet.

**Claims**

1. Method for packing parenteralia in transparent soft plastics containers suitable for steam sterilization, wherein the parenteralia are first filled into an inner container of transparent soft plastic and then the inner container is closed and sealed in a protective wrap of transparent soft plastic being impermeable for microorganisms and having a high water vapor blocking effect, characterized in that the filled and closed inner container is laid in a lower web of the protective wrap which has been deep-drawn in accordance with the contours of the inner container, and is covered with the upper web of the protective wrap, a vacuum is applied to the protective wrap and the edges of the lower and upper webs of the protective wrap are welded together.

2. Method according to claim 1, characterized in that before or during sealing of the protective wrap a vacuum of from about 500 to 600 mbar is applied.

**Revendications**

1. Procédé pour emballer des produits injectables par voie parentérale dans des récipients transparents stérilisables à la vapeur d'eau faits de matières synthétiques molles, dans lequel on transvase d'abord les produits injectables par voie parentérale dans un récipient intérieur en matière synthétique molle et transparente puis on ferme le récipient intérieur et on le scelle dans une enveloppe protectrice faite d'une matière synthétique transparente, molle, imperméable aux microorganismes et ayant une étanchéité élevée à la vapeur d'eau, caractérisé en ce qu'on dispose le récipient intérieur rempli et fermé dans un lé inférieur de l'enveloppe protectrice embouti de façon correspondant à ses contours, en ce qu'on le recouvre avec le lé supérieur de l'enveloppe protectrice, en ce qu'on applique un vide à l'enveloppe protectrice et en ce qu'on scelle les uns aux autres les bords du lé inférieur et du lé supérieur de l'enveloppe protectrice.

2. Procédé selon la revendication 1, caractérisé en ce qu'on applique avant ou pendant le scellement de l'enveloppe protectrice un vide d'environ 500 à 600 mbar.